# EUROPEAN PATENT APPLICATION

(11) **EP 2 447 865 A1**
(43) Date of publication of application: **02.05.2012**
(21) Application number: 10014109.2
(22) Date of filing: 29.10.2010
(51) Int. Cl.: G06F 19/00

(54) **Molecular field analysis based on cosmo polarization densities**

(71) Applicant: COSMOlogic GmbH & Co. KG, 51381 Leverkusen (DE)
(72) Inventor: Klamt, Andreas, Dr., 51381 Leverkusen (DE); Thormann, Michael, Dr., 82152 Planegg (DE)
(74) Representative: Patentanwälte Westphal, Mussgnug & Partner

(57) **Abstract**

The invention relates to a computer-based method for generating a three-dimensional quantitative structure-activity relationship comprising the steps of a) providing a set of molecules with known binding constants or physiological activity data and information of the structure of said molecules, b) aligning the molecules to achieve maximum local similarity of molecular interaction regions that are common to the set of molecules, c) providing a surface polarity descriptor of the molecular surface for the set of molecules d) projecting the surface polarity descriptor to a fixed three-dimensional grid, wherein the same three-dimensional grid is used for all molecules and wherein the projection involves the use of local surface property histograms, mathematical equivalents thereof or derivatives thereof, and e) performing a molecular field analysis or an equivalent statistical analysis in order to derive a predictive model for the binding constant or physiological activity, wherein the molecular field analysis or the equivalent statistical analysis are at least partly based on the projection of the surface polarity descriptor to the three-dimensional grid.

## Description

Pharmaceutical drug design is mostly governed by application of the lock-and-key principle. The receptor that is being targeted is the lock, and the group occupying said receptor, i.e. the drug or a ligand, is the key.

In general, the task to be solved in drug design is the development of better keys. E.g., a drug that binds more selectively to a given receptor may have reduced side-effects compared to a drug with a less specific binding behavior. Obviously, the chance of developing a better key (i.e. a better acting drug or an alternative, patent-free drug) improves drastically with the information on the lock that is available.

Currently, there are two main approaches that are being followed to provide this type of information. On the one hand, structure-based drug design is applied, which relies on structural information obtained mainly by means of protein crystallography. In spite of significant increase in the throughput capacity of facilities which provide the possibility to obtain these structural data, obtaining as detailed information on protein structure as required for successful drug design is still expensive in terms of both time and money.

Therefore, as an alternative or in addition to protein crystallographic methods, the "active analog" approach is applied. This approach is based on the concept that general chemical and stereochemical aspects also govern biochemical interactions, so that it is reasonable to assume that variations in biological activity within a set of compounds can be explained by variations in their molecular property descriptors, which in turn are local, so that properties of ligands are assumed to play a dominant role. Consequently, the comparison of molecular property descriptors within a set of molecules whose biological activities are well known should be suited for the determination of the molecular properties relevant for a given biological activity, so that one should look specifically for other molecules that show this molecular property and thus may be suitable candidates for this type of biological activity.

One of the most popular realizations of the "active analog" approach is known in the field as "CoMFA" (Comparative Molecular Field Analysis) and disclosed e.g. in EP 0 592 421 B1. In this method, steric and electrostatic interaction fields are calculated as descriptors for a set of ligands or molecules with known biological properties on grid points of a rectangular box that is of bigger size than the largest molecule in the set. To determine the interaction field, probes are used, e.g. an sp³-hybridized Carbon atom for steric interaction and a positive point charge for Coulomb interaction.

However, experience with the existing methods indicates that they suffer from several drawbacks. Insufficient local resolution of the known descriptors for molecular field analysis in general and specifically for CoMFA lead to an insufficient level of detail and a description that is not as local as one would like even if small grid spacings are used. This leads to the problem of providing a more local and detailed molecular descriptor for molecular field analysis methods that can be applied even at relatively large grid spacings of 1x10⁻¹⁰ m to 2x10⁻¹⁰ m.

This problem is solved by a computer-based method with the features of claim 1.

The computer-based method for generating a three-dimensional quantitative structure-activity relationship between a set of molecules and a target molecule or probe atom comprises at least the following steps, preferably performed in this order but potentially including further intermediate steps:
a) providing a set of molecules with known binding constants or physiological activity data and information of the structure of said molecules. This set of molecules essentially forms a training set that provides the information basis based on which the relevance of a given structural feature or combination of structural features can be obtained. Proper selection of the compounds included in this training set is crucial for the successful establishment of a valid quantitative structure-activity relationship.
b) aligning the molecules to achieve maximum local similarity of molecular interaction regions that are common to the set of molecules. This step is essential to ensure that in fact molecular interaction groups of the training set of molecules that interact with the same site of the target are compared to each other in the determination of the respective effect of a given group. Suitable alignment tools are known to a person skilled in the art, e.g. COSMOsim3D can be used to perform this step of the method.
c) providing a surface polarity descriptor of the molecular surface for the set of molecules. This is done using computational chemistry calculations known to a person skilled in the art, e.g. DFT-COSMO calculations. Suitable surface polarity descriptors are e.g. the surface polarization charge density σ and entities related to or derived from σ. In other words, the surface polarization charge density may directly be used as calculated from COSMO or COSMO related algorithms as implemented e.g. in TURBOMOLE, Gaussian03, DMOL or other quantum chemistry suites. Alternatively, a related property, e.g. an electrostatic potential directly calculated on a virtual molecule surface, or a derived property as obtained e.g. by using smeared out charges or statistically modified, especially averaged charges may be used.
d) projecting the surface polarity descriptor to a fixed three-dimensional grid, wherein the same three-dimensional grid is used for all molecules and wherein the projection involves the use of local surface property histograms, mathematical equivalents thereof or derivatives thereof. This step, more specifically the use of local surface property histograms or related entities in connection with the use of a surface polarity descriptor, is essential for this invention, because per se these descriptors, especially the surface polarization charge density σ and derivations thereof, are an entity that comprises 2D-surface-information only and not the 3D-information required for descriptors in molecular field analysis methods. Only the projection step d) transforms the 2D-surface-information to information that may be used for molecular field analysis.

At the same time, surface polarity descriptors like the surface polarization charge density σ and derivations thereof, and the local histograms thereof that are made accessible for molecular field analysis methods by this invention contain all relevant information with respect not only to electrostatic information and steric requirements, but also to hydrogen bond interactions and hydrophobic interactions included in a single property without the need to use separate fields for these properties, which in turn need to be weighted appropriately in order to achieve correct results.

It has been discovered by the inventors that surface polarity descriptors and especially σ can actually provide a more detailed and more local description of the interactions compared to other properties used for molecular field analysis when set up as a descriptor as claimed in step d). In additionto the wealth of highly local information contained in a single value of σ, the use of local histograms of said entity allows for maintaining a high level of local detail even at larger grid spacings of 1x10⁻¹⁰ m to 2x10⁻¹⁰ m, which in turn leads on the one hand to the technical advantage of using a smaller amount of data which simplifies statistical analysis and on the other hand represents the typical flexibility of ligands and receptors and thus corresponds to the physical situation in a better way.
e) performing a molecular field analysis or an equivalent statistical analysis in order to derive a predictive model for the binding constant or physiological activity, wherein the molecular field analysis or the equivalent statistical analysis are at least partly based on the projection of the surface polarity descriptor to the three-dimensional grid. This predictive model is the key information needed and applied for the development of new and/or improved drugs, as it identifies candidates for the biological activity one wishes to achieve that may then be tested for their suitability.

In an advantageous embodiment, step c) is performed before step b) and the alignment is at least partly based on the surface polarity descriptor. In this way, a very precise alignment can be obtained. It is advantageous if the alignment is based on the surface polarity descriptor and at least one of the group consisting of geometry information and element information.

Another increase in predictive power of the method is achieved if the molecular field analysis or an equivalent statistical analysis performed in step e) is based on the surface polarity descriptor and at least one of the group consisting of geometry information and element information.

In a preferred embodiment of the invention, the surface polarity descriptor is the molecular surface polarization charge density σ or comprises the molecular surface polarization charge density σ.

Specifically, the use of the computer based method as claimed for rational drug design (drug targeting) is advantageous.

### Brief description of drawings:

The figures show:
   - Fig. 1: a block diagram illustrating the essential steps of the method of this invention,
   - Fig. 2a: a simplified example for illustrating the concept of histograms of a surface polarity descriptor, and
   - Fig. 2b: the histogram corresponding to the example of Fig. 2a.

### Detailed description of the invention:

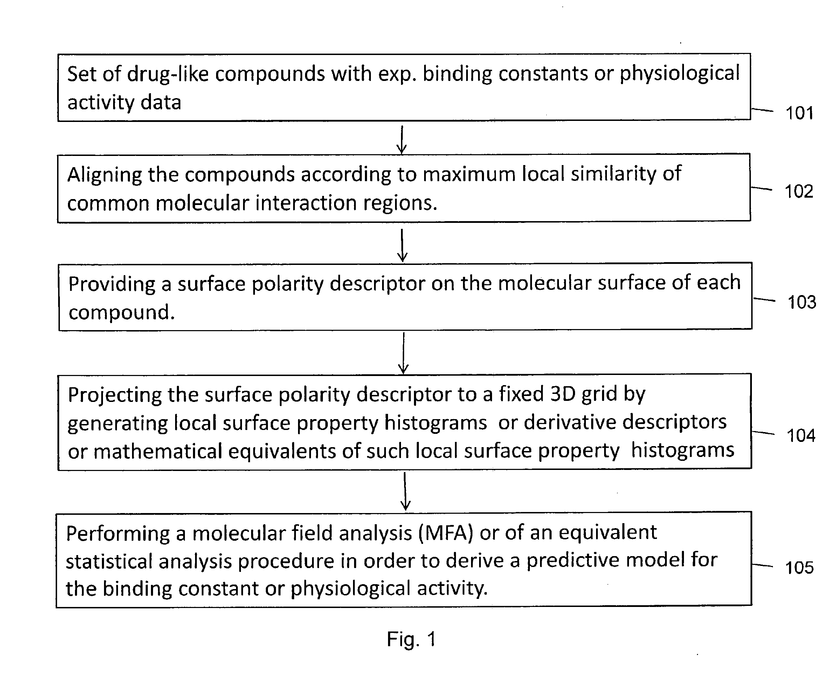

Figure 1 shows a block diagram illustrating the essential steps of one embodiment of the method of this invention.

In the first step 101 of the method, a set of molecules with known binding constants or physiological activity data are chosen, which form the information basis for the structure-activity relationship or a training set. In addition, it is necessary to provide information about the structure of the molecule, which can be obtained for example from a crystallographic database like the Cambridge Crystallographic Database or determined using standard molecular modeling techniques like force field methods like AMBER or GROMOS, as implemented in commonly available simulation packages, or quantum chemical methods like DFT or HF calculations as implemented in TURBOMOLE, DMOL, Gaussian03 and many other quantum chemical software suites.

In step 102 of the method, an alignment of the molecules takes place. This alignment aims at an orientation of the molecules in such a way that maximum local similarity of common molecular interaction regions is achieved. It can be done e.g. using the COSMOsim 3D tool, but also using any other alignment tool. In step 103 of the method, a surface polarity descriptor of the molecular surface for the set of molecules is provided. This can be done e.g. by running DFT-COSMO calculations for all compounds.

It should be noted that it may be useful to perform at least part of the calculations for providing the surface polarity descriptor before the alignment takes place because said surface polarity descriptor may also be used in connection with a suitable similarity measure for the alignment performed in step 102.

Step 104 of the method is a key step for this invention. In this step, the surface polarity descriptor is projected onto a fixed three-dimensional grid, wherein the same three-dimensional grid is used for all molecules.

Apart from the example, the grid does not have to be cubic or rectangular and can have any shape or design. A favorable grid can be designed especially around the molecular surface or have increased resolutions at important surface pieces of the molecule. In this way, increased accuracy can be achieved with the same or less computational demand as in a simple cubic grid.

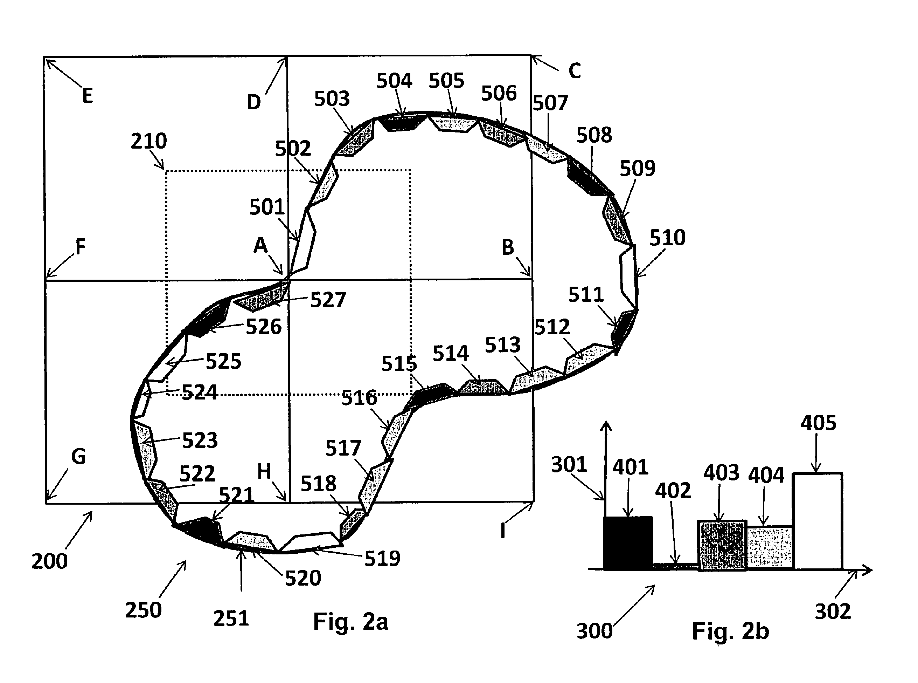

The projection involves the use of local surface property histograms or mathematical equivalents or derivatives thereof. To clarify the meaning of the use of local surface property histograms, in figures 2a and 2b a simplified example is provided for the purpose of illustrating this concept.

Relating to figure 2a, this figure shows part of a grid plane 200 formed by central grid point A and grid points B, C, D, E, F, G, H, I that belongs to a three-dimensional grid and the line 251 representing the cut through an aligned molecule 250 lying in said plane. It illustrates in a simplified way how the histogram 300 shown in Figure 2b of the surface polarity descriptor for the central grid point A is calculated.

In step 103 of the method, the surface polarity descriptor has been calculated for the molecule, which means that the molecule surface has been divided into a number of patches for which a value of the surface polarity descriptor is known.

Figure 2a shows the patches 501-527 that lie on the line 251 representing the part of the three-dimensional surface of the aligned molecule 250. In this example, the values of the surface polarity descriptor for each patch 501-527 are binned into five bins 401 - 405 or groups and each of these bins 401 - 405 or groups is assigned a color coding. Naturally, the number of bins 401 - 405 may be varied and is not limited by the value provided in this example.

E.g., if the surface polarity descriptor could take values between 0 and 99, the binning and color coding could mean that patches with surface polarity descriptor values between
- 0 and 19 are assigned to a first bin 401 and colored black,
- 20 and 39 are assigned to a second bin 402 and colored dark grey,
- 40 and 59 are assigned to a third bin 403 and colored medium grey,
- 60 and 79 are assigned to a fourth bin 404 and colored light grey, and
- 80 and 99 are assigned to a fifth bin 405 and colored
   white.

Correspondingly, in this example,
- the first bin 401 would comprise patches 504,508,511,515, 521 and 526,
- the second bin 402 would comprise no patches,
- the third bin 403 would comprise patches 503, 506, 509, 514, 518, 522 and 527,
- the fourth bin 404 would comprise patches 502, 505, 507, 512, 513, 516, 517, 520 and 523, and
- the fifth bin 405 would comprise patches 501, 510, 519, 524 and 525.

Note that in general as illustrated in figure 2a, the number of grid points A - I may be considerably smaller than the number of patches 501-526. This allows for the inclusion of highly local information into a rather coarse grid.

The histogram shown in Figure 2b can be obtained by simply evaluating the area of the patches assigned to a given bin inside a catchment area 210 marked in figure 2a by a dotted line and displaying the corresponding amount of surface area on histogram axis 302 versus the value of the surface polarity descriptor that is binned into bins 401 - 405 on histogram axis 301. In this specific example, inside the catchment area patch 526 belongs to the first bin 401, patch 527 belongs to the third bin 403, patch 502 belongs to the fourth bin 404 and patches 501 and 525 belong to the fifth bin 405. Note that in spite of an identical number of patches belonging to a given bin 401 - 405, the surface area represented by said patches may vary if the patches are not equal in size, as illustrated in figure 2b.

The most important simplification made in figure 2a for the purpose of illustration resides in the fact that the dimensions involved have been reduced. In reality, the molecular surface descriptor is calculated and evaluated on a two-dimensional surface rather than the one-dimensional line of figure 2a. Consequently, in reality the creation of the histograms involves a catchment volume in the full grid rather than a catchment area in the histograms that are considered. Relating again to figure 1, as step 105 of the method a molecular field analysis or an equivalent statistical analysis is performed in order to derive a predictive model for the binding constant or physiological activity based on which a new and/or improved drug can be developed. The molecular field analysis or the equivalent statistical analysis is at least partly based on the projection of the surface polarity descriptor to the three-dimensional grid. Specifically, the data points of the histogram could be directly used in common MFA-approaches, as each bin of the histogram can replace one of the multiple descriptors of standard MFA techniques. Alternatively, a reduced or derived histogram such as slopes, integrals, moments or correlated regions can be used to increase numerical stability and physical meaning of the descriptor.

### Reference numerals

- 101-105: method steps
- 200: grid plane
- 210: catchment area
- 250: molecule
- 251: line
- 300: histogram
- 301,302: histogram axis
- 401-405: bins
- 501-527: patches

- A, B, C, D, E, F, G, H, I: grid points

## Claims

1. A computer-based method for generating a three-dimensional quantitative structure-activity relationship comprising the steps of
a) providing a set of molecules with known binding constants or physiological activity data and information of the structure of said molecules,
b) aligning the molecules to achieve maximum local similarity of molecular interaction regions that are common to the set of molecules,
c) providing a surface polarity descriptor of the molecular surface for the set of molecules
d) projecting the surface polarity descriptor to a fixed three-dimensional grid, wherein the same three-dimensional grid is used for all molecules and wherein the projection involves the use of local surface property histograms, mathematical equivalents thereof or derivatives thereof, and
e) performing a molecular field analysis or an equivalent statistical analysis in order to derive a predictive model for the binding constant or physiological activity, wherein the molecular field analysis or the equivalent statistical analysis are at least partly based on the projection of the surface polarity descriptor to the three-dimensional grid.

2. Computer based method as claimed in claim 1,
**characterized in that** at step c) is performed before step b) and that the alignment is at least partly based on the surface polarity descriptor.

3. Computer based method as claimed in claim 2,
**characterized in that** the alignment is based on the surface polarity descriptor and at least one of the group consisting of geometry information, element information and electrostatic potential.

4. Computer based method as claimed in one of claims 1 to 3, **characterized in that** the molecular field analysis or an equivalent statistical analysis performed in step e) is based on the surface polarity descriptor and at least one of the group consisting of geometry information, element information and electrostatic potential.

5. Computer based method as claimed in one of claims 1 to 4, **characterized in that** the surface polarity descriptor is the molecular surface polarization charge density σ or comprises the molecular surface polarization charge density σ.

6. Use of the computer based method as claimed in one of claims 1 to 5 for rational drug design.

## Amended claims

### Amended claims in accordance with Rule 137(2) EPC.

**1.** A computer-based method for generating a three-dimensional quantitative structure-activity relationship comprising the steps of
a) providing a set of molecules with known binding constants or physiological activity data and information of the structure of said molecules,
b) aligning the molecules to achieve maximum local similarity of molecular interaction regions that are common to the set of molecules,
c) providing a surface polarity descriptor of the molecular surface for the set of molecules
d) projecting the surface polarity descriptor to a fixed three-dimensional grid, wherein the same three-dimensional grid is used for all molecules and wherein the projection involves the use of local surface property histograms, mathematical equivalents thereof or derivatives thereof, and
e) performing a molecular field analysis or an equivalent statistical analysis in order to derive a predictive model for the binding constant or physiological activity, wherein the molecular field analysis or the equivalent statistical analysis are at least partly based on the projection of the surface polarity descriptor to the three-dimensional grid.

**2.** Computer based method as claimed in claim 1,
**characterized in that** at step c) is performed before step b) and that the alignment is at least partly based on the surface polarity descriptor.

**3.** Computer based method as claimed in claim 2,
**characterized in that** the alignment is based on the surface polarity descriptor and at least one of the group consisting of geometry information, element information and electrostatic potential.

**4.** Computer based method as claimed in one of claims 1 to 3,
**characterized in that** the molecular field analysis or an equivalent statistical analysis performed in step e) is based on the surface polarity descriptor and at least one of the group consisting of geometry information, element information and electrostatic potential.

**5.** Computer based method as claimed in one of claims 1 to 4,
**characterized in that** the surface polarity descriptor is the molecular surface polarization charge density σ or comprises the molecular surface polarization charge density σ.
